# EUROPÄISCHE PATENTSCHRIFT

(11) **EP 3 002 033 B1**
(45) Veröffentlichungstag und Bekanntmachung des Hinweises auf die Patenterteilung: **04.09.2019**
(21) Anmeldenummer: 15184194.7
(22) Anmeldetag: 08.09.2015
(51) Int. Cl.: A61M 39/10

(54) **VERBINDUNGSVORRICHTUNG FÜR EIN FLUIDSYSTEM FÜR MEDIZINISCHE ZWECKE**
CONNECTING DEVICE FOR A FLUID SYSTEM FOR MEDICAL PURPOSES
DISPOSITIF DE CONNEXION D'UN SYSTEME FLUIDIQUE A DES FINS MEDICALES

(30) Priorität: 30.09.2014 DE 102014219814
(43) Veröffentlichungstag der Anmeldung: 06.04.2016
(73) Patentinhaber: B. Braun Melsungen AG, 34212 Melsungen (DE)
(72) Erfinder: BOLZ, Johannes, 34132 Kassel (DE); JÖRN, Jens, 34130 Kassel (DE); KATERKAMP, Andreas, 34212 Melsungen (DE)
(74) Vertreter: Patentanwälte Ruff, Wilhelm, Beier, Dauster & Partner mbB

(56) Entgegenhaltungen:
- EP-A1- 2 810 685
- WO-A1-96/40359
- WO-A2-2008/043069
- DE-A1-102007 020 859
- DE-A1-102008 034 919
- US-A- 3 797 486
- US-A- 4 911 705
- US-A- 5 098 405

## Beschreibung

Die Erfindung betrifft eine Verbindungsvorrichtung für ein Fluidsystem für medizinische Zwecke mit einem Grundgehäuse, das einen Fluidströmungsraum aufweist, der in verschiedenen Richtungen zu zwei offenen Anschlüssen hin führende Kanalabschnitte ausbildet, wobei die Anschlüsse zur Verbindung mit jeweils einer Fluidleitung oder einem Fluidspeicher vorgesehen sind, sowie mit einem in den Fluidströmungsraum mündenden Anschlussport, der mit einer Verschlussanordnung versehen ist, und der einen Verbindungsabschnitt zur Verbindung mit einem Anschlussteil umfasst, wobei der Fluidströmungsraum eine formstabile Strömungsleitwand aufweist, die zwischen den beiden Kanalabschnitten im Fluidströmungsraum starr angeordnet ist.

Eine derartige Verbindungsvorrichtung ist aus der WO 2008/043069 A2 bekannt. Die bekannte Verbindungsvorrichtung weist eine Strömungsleitwand auf, die dazu dient, stehende Flüssigkeit in einem Strömungsraum mitzunehmen, sobald Fluid in einen Y-Anschluss eingespritzt wird.

Die US 4,911,705 A offenbart eine weitere Verbindungsvorrichtung für ein Fluidsystem für medizinische Zwecke, bei der eine Strömungsleitwand dazu vorgesehen ist, stehende Flüssigkeit im Bereich einer Ventilmembran mitzunehmen, sobald in einen Y-Anschluss Flüssigkeit eingespritzt wird.

Eine weitere Verbindungsvorrichtung ist aus der US 7,931,627 B2 bekannt. Die Verbindungsvorrichtung ist als Y-Port für ein medizinisches Infusionssystem vorgesehen. In einem Grundgehäuse ist ein Fluidströmungsraum angeordnet, der mehrere Kanalabschnitte ausbildet. Zwei Kanalabschnitte zweigen zu unterschiedlichen Richtungen hin zu jeweils einem offenen Anschluss ab, der jeweils mit einer Schlauchleitung verbindbar ist, die zu einem Katheter bzw. zu einem Fluidspeicherbehältnis führt. Zudem weist das Grundgehäuse einen in Verlängerung des einen Kanalabschnitts zu einer entgegengesetzten Richtung hin offenen Anschlussport auf, der einen Luer-Verbindungsabschnitt umfasst, um ein entsprechendes Luer-Anschlussteil wie eine Spritze oder ein Fluidspeicherbehältnis anschließen zu können. In dem Fluidströmungsraum ist eine flexible Rückschlagklappe vorgesehen, die den einen Kanalabschnitt am Abzweig zum anderen Kanalabschnitt innerhalb des Fluidströmungsraums verschließen kann. Dabei erfolgt durch die flexible, laschenartige Klappe keine vollständige Absperrung dieses Kanalabschnitts, sondern lediglich eine Reduzierung der Durchströmung des Kanalabschnitts.

Aufgabe der Erfindung ist es, eine Verbindungsvorrichtung der eingangs genannten Art zu schaffen, die einfacher aufgebaut ist und eine kontaminationsarme, sichere Handhabung ermöglicht.

Diese Aufgabe wird durch die Merkmale des Anspruchs 1 gelöst. Ein Fokussieren der Fluidströmung aus dem Anschlussport auf den einen Kanalabschnitt erfolgt erfindungsgemäß durch Zusammenwirken der sich öffnenden Verschlussanordnung mit einem Teilabschnitt der Strömungsleitwand. Vorzugsweise wird die Verschlussanordnung durch eine elastisch flexible Verschlussmembran in Verbindung mit einem beweglichen Betätigungskörper gebildet, der zum Öffnen der Verschlussmembran linearbeweglich angeordnet ist. Die Strömungsleitwand ist starr im Fluidströmungsraum angeordnet, wobei sie bei geschlossener Verschlussmembran eine entsprechende Fluidströmung zwischen dem einen und dem anderen Kanalabschnitt ermöglicht und gegebenenfalls umlenkt. Die formstabile Strömungsleitwand ermöglicht eine definierte und gleichbleibende Fluidströmung durch den Fluidströmungsraum hindurch, solange die Verschlussanordnung den Anschlussport absperrt. Ein separates Rückschlagventil oder eine Klemme in einer Fluidleitung zu dem Anschluss des einen Kanalabschnitts wird daher erfindungsgemäß nicht benötigt. Die erfindungsgemäße Lösung eignet sich in besonders vorteilhafter Weise für einen Y-Port innerhalb eines medizinischen Infusions- oder Transfusionssystems, wobei vorzugsweise ein Anschluss des einen Kanalabschnitts in eine Patientenleitung zu einem Katheter fortführt, und der Anschluss des anderen Kanalabschnitts zu einem Fluidspeicherbehältnis führt, in dem eine medizinische Infusionslösung gespeichert ist, die dem Patienten über den Y-Port und die Patientenleitung zugeführt wird. Der Anschlussport der so eingesetzten Verbindungsvorrichtung dient vorzugsweise dazu, je nach Bedarf dem Patienten ergänzend noch hochwirksame Medikamente mit hochdosierten medizinischen Wirkstoffen zuzuführen. Als geeignetes Anschlussteil kann eine mit einer Luer-Lock- oder einer Luer-Slip-Verbindung versehene Infusionsspritze vorgesehen sein, die mit dem korrespondierenden Verbindungsabschnitt des Anschlussports in einfacher Weise manuell verbunden und wieder gelöst werden kann. Dabei wird zwangsläufig die Verschlussanordnung geöffnet bzw. wieder verschlossen. Alternativ ist der Verbindungsabschnitt für enterale Anwendungen als Enteral Small Bore Connector ausgebildet, der einen enteralen Verbindungsabschnitt bildet, der inkompatibel mit nicht-enteralen Anschlussabschnitten ist. Dadurch wird verhindert, dass enterale Funktionsteile mit nicht-enteralen Funktionsteilen verbunden werden können.

In vorteilhafter Weise ist der Anschlussport koaxial fluchtend zu dem Kanalabschnitt des Grundgehäuses ausgerichtet, in den das durch den Anschlussport zugeführte Fluid weitergeleitet werden soll. Der andere Kanalabschnitt hingegen ist vorzugsweise nach Art eines Y-Abzweigs winklig zu dem ersten Kanalabschnitt im Grundgehäuse ausgerichtet. Die Anschlüsse des Grundgehäuses, in die die beiden Kanalabschnitte nach außen münden, können mit geeigneten Konnektoren versehen sein, um die Anbindung einer entsprechenden Schlauchleitung oder eines Anschlussstutzens eines Fluidspeicherbehältnisses oder eines anderen Verbindungsteils zu vereinfachen. Der bewegliche Betätigungskörper kann in seiner Öffnungsstellung knapp oberhalb eines als Teilabschnitt dienenden Stirnrandes der Strömungsleitwand oder auch in Anlage auf dem Stirnrand positioniert sein. Alternativ können sich der bewegliche Betätigungskörper oder die flexible Verschlussmembran in der Öffnungsstellung seitlich an einen entsprechenden Teilabschnitt der Strömungsleitwand anschmiegen, um so die über den Anschlussport zugeführte Fluidströmung in den Kanalabschnitt weiterzuleiten, der mit dem Anschlussport fluchtet.

Erfindungsgemäß ist in dem Anschlussport als Teil der Verschlussanordnung ein beweglicher Betätigungskörper zum Öffnen einer flexiblen Verschlussmembran in Abhängigkeit von einer Verbindung mit einem Luer-Anschlussteil gelagert. Dadurch ist sichergestellt, dass die Verschlussmembran beim Anschließen eines Luer-Anschlussteils an den Anschlussport zuverlässig öffnet. Die Verschlussmembran ist aus einem elastisch flexiblen Kunststoffmaterial wie insbesondere Silikon hergestellt und geschlitzt, so dass sie sich bei elastischer Deformation öffnet. Der Betätigungskörper ist zudem mit einem Durchtrittskanal versehen, der aufgrund seines Strömungsquerschnitts eine sichere und gleichbleibende Zuströmung eines medizinischen Fluids aus dem Luer-Anschlussteil in den Fluidströmungsraum hinein gewährleistet.

Erfindungsgemäß ragt die Störmungsleitwand derart in Richtung des Anschlussports ab, dass der Betätigungskörper und/oder die flexible Verschlussmembran in der Öffnungsstellung der Verschlussanordnung mit Teilbereichen der Strömungsleitwand derart in Anlage sind, dass eine in den Anschlussport von dem Luer-Anschlussteil zugeführte Fluidströmung zumindest weitgehend in den Kanalabschnitt strömt. Dabei können entsprechende Wandungs- oder Flächenabschnitte des Betätigungskörpers oder der Verschlussmembran mit korrespondierenden Teilbereichen der Strömungsleitwand in der Öffnungsstellung in Kontakt sein. Entsprechende Teilbereiche der Strömungsleitwand sind Stirnwandbereiche oder seitliche Wandungsbereiche, so dass der Betätigungskörper und/oder die flexible Verschlussmembran in der Öffnungsstellung stirnseitig der Strömungsleitwand oder seitlich neben der Störmungsleitwand positioniert sein können.

Erfindungsgemäß sind der Betätigungskörper und/oder die flexible Verschlussmembran in der Öffnungsstellung der Verschlussanordnung auf oder neben wenigstens einem Teilbereich der Strömungsleitwand positioniert. Der Begriff der Positionierung ist so zu verstehen, dass der Betätigungskörper und/oder die flexible Verschlussmembran entweder in geringem Abstand auf oder neben dem entsprechenden Teilbereich der Strömungsleitwand, oder in entsprechender Anlage auf oder neben dem Teilbereich der Strömungsleitwand angeordnet sind.

In Ausgestaltung der Erfindung ist die Strömungsleitwand einstückig in dem Grundgehäuse integriert. Die Strömungsleitwand wird vorzugsweise bei der Herstellung des Grundgehäuses aus einem geeigneten Kunststoffmaterial einstückig mit ausgeformt.

In weiterer Ausgestaltung der Erfindung ist die Strömungsleitwand an dem freien Stirnrand über einen Teilbereich koaxial zu einer Mittellängsachse des Anschlussports im Querschnitt bogen- oder V-artig gekrümmt, wobei die Strömungsleitwand in einem Mündungsbereich des einen Kanalabschnitts in den anderen Kanalabschnitt angeformt ist. Der freie Stirnrand kommt zumindest abschnittsweise mit der Verschlussanordnung in Anlage, sobald die Verschlussanordnung geöffnet wird und sich in Richtung des Fluidströmungsraums hinein verformt/verlagert. Die Strömungsleitwand bildet demzufolge eine Rinne, die koaxial oder achsparallel zu dem Kanalabschnitt verläuft, in dem das durch das Luer-Anschlussteil zugeführte Fluid weitergeleitet werden soll.

In weiterer Ausgestaltung der Erfindung ist eine Krümmung des freien Stirnrands derart auf einen zu der Mittellängsachse des Anschlussports koaxialen Durchtrittskanal des Betätigungskörpers abgestimmt, dass eine derart gebildete Rinne der Strömungsleitwand auf einer dem einen Kanalabschnitt zugewandten Seite bei in seine Öffnungsstellung verlagertem Betätigungskörper mit dem Durchtrittskanal des Betätigungskörpers fluchtet. Der Betätigungskörper kann mit seiner dem Stirnrand der Strömungsleitwand zugewandten Stirnfläche auf diesem aufliegen oder in geringem Abstand zu diesem positioniert sein, wenn der Betätigungskörper in die Öffnungsstellung verlagert ist. So bleibt die Verbindung zwischen dem Durchtrittskanal und dem anderen Kanalabschnitt offen, um das gewünschte Zuspritzen eines Medikaments durch den Anschlussport hindurch zu dem Kanalabschnitt und damit insbesondere zur Patientenleitung zu ermöglichen.

In weiterer Ausgestaltung der Erfindung ist die Strömungsleitwand für den einen Kanalabschnitt als einen Fluidstrom umlenkende Strömungsleitfläche ausgeführt, die eine Fluidumlenkung in Richtung des Anschlussports von aus dem einen Kanalabschnitt in Richtung des anderen Kanalabschnitts strömendem Fluid bewirkt. Die durch die Strömungsleitwand gebildete Strömungsleitfläche ermöglicht bei geschlossener Verschlussanordnung eine definierte und gleichbleibende Strömungsumlenkung des entsprechenden medizinischen Fluids. Die Strömungsquerschnitte im Bereich des einen Kanalabschnitts, im Bereich des anderen Kanalabschnitts sowie im Bereich der Umlenkung durch die Strömungsleitwand sind bezüglich ihrer Strömungsfläche vorzugsweise identisch, so dass ein gleichbleibender Fluidstrom durch das Fluidsystem fließen kann.

Die der Erfindung zugrundeliegende Aufgabe wird auch dadurch gelöst, dass der Anschlussport in einem zu dem Grundgehäuse separat hergestellten Gehäusebauteil vorgesehen ist, das aus einem inerteren Kunststoffmaterial hergestellt ist als ein Kunststoffmaterial des Grundgehäuses, um eine höhere chemische Beständigkeit als das Grundgehäuse gegenüber insbesondere hochdosierten medizinischen Wirkstoffen zu erzielen, die am Anschlussport dem Fluidsystem zuführbar sind. Dadurch wird eine Kontamination des Anschlussports und insbesondere eine Reaktion des Kunststoffmaterials des Anschlussports mit entsprechend zugeführten, toxischen medizinischen Wirkstoffen vermieden, so dass der Anschlussport zuverlässig eingesetzt werden kann, um mehrfach unterschiedliche Arten von medizinischen Wirkstoffen dem Fluidsystem und insbesondere einer Patientenleitung zugeben zu können. Erfindungsgemäß geeignete inerte Materialien für das Gehäusebauteil sind insbesondere Polyethylen (PE), Polypropylen (PP), Polysulfon (PSE) oder Copolyester. Geeignete Kunststoffe für das Grundgehäuse sind Polycarbonat (PC) oder Acrylnitril-Butadien-Styrol (ABS).

In weiterer Ausgestaltung der Erfindung ist das den Anschlussport aufweisende Gehäusebauteil stoffschlüssig, insbesondere durch Verklebung oder Verschweißung, mit dem Grundgehäuse dicht verbunden. In besonders vorteilhafter Weise wird zur Verklebung ein durch UV-Strahlung aushärtender Klebstoff eingesetzt.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1a: zeigt in aufgeschnittener, perspektivischer Explosionsdarstellung eine Ausfüh-rungsform einer erfindungsgemäßen Verbindungsvorrichtung in Form eines Y-Ports,
- Fig. 1b: die Verbindungsvorrichtung nach Fig. 1a in einer Seitenansicht,
- Fig. 2: einen Längsschnitt durch die Verbindungsvorrichtung nach Fig. 1 in einer Schließstellung einer flexiblen Verschlussmembran,
- Fig. 3: die Verbindungsvorrichtung nach Fig. 2 in einer Öffnungsstellung der flexiblen Verschlussmembran und
- Fig. 4: in vergrößerter Querschnittsdarstellung einen Schnitt durch die Verbindungsvorrichtung nach den Fig. 1 bis 3 entlang der Schnittlinie IV-IV in Fig. 3.

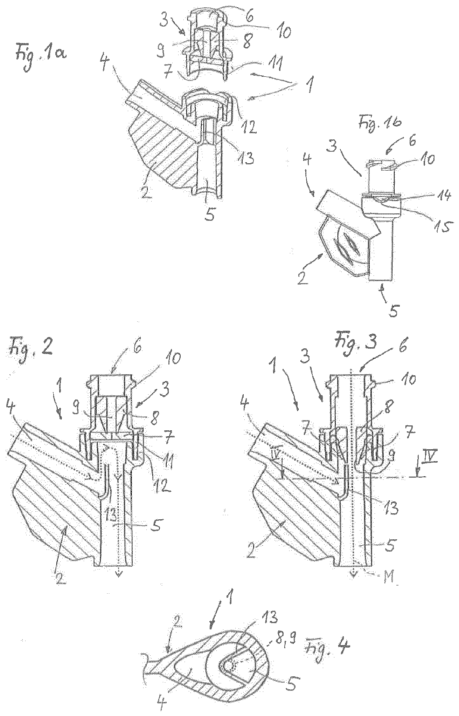

Eine Verbindungsvorrichtung nach den Fig. 1 bis 4 ist für den Einsatz bei einem intravenösen Infusionssystem für medizinische Zwecke vorgesehen. Die Verbindungsvorrichtung 1 ist als sogenannter Y-Port ausgeführt, der insgesamt drei nachfolgend näher beschriebene Anschlüsse zur Verfügung stellt, die innerhalb der Verbindungsvorrichtung 1 einander zu einem Fluidströmungsraum vereinigen, der Y-artig verzweigte Kanalabschnitte 4, 5 umfasst. Der durch die Kanalabschnitte 4, 5 gebildete Fluidströmungsraum ist innerhalb eines Grundgehäuses 2 angeordnet, das aus Kunststoff einteilig hergestellt ist. Der erste, anhand der Fig. 1 bis 3 sich schräg nach links oben erstreckende Kanalabschnitt 4 weist an seinem äußeren Stirnendbereich einen Anschluss für die Verbindung des Grundgehäuses 2 mit einem Fluidspeicher wie insbesondere einer Flasche oder einem Beutel auf, die eine geeignete medizinische Infusionslösung speichern. Der in den Fig. 1 bis 3 nach unten zeigende Kanalabschnitt 5 weist im Bereich seines unteren Stirnendes einen Anschluss für die Verbindung mit einer als Patientenleitung dienenden Schlauchleitung auf, die zu einem Katheter führt, der dem Patienten intravenös zuführbar ist.

Um insbesondere dem Patienten ergänzend Medikamente in Form von insbesondere hochdosierten medizinischen Wirkstoffen zuführen zu können, weist die Verbindungsvorrichtung 1 ergänzend einen Anschlussport 6 auf, der in koaxialer Verlängerung des Kanalabschnitts 5 gemäß den Darstellungen nach den Fig. 1 bis 3 von oben her auf dem Grundgehäuse 2 angeordnet ist. Der Anschlussport 6 wird durch ein separates Kunststoffbauteil 3 gebildet, das aus einem gegenüber dem Kunststoffmaterial des Grundgehäuses 2 inerteren Kunststoff besteht. Das separat hergestellte Kunststoffbauteil 3 weist demzufolge eine höhere chemische Beständigkeit gegenüber medizinischen Wirkstoffen auf, die über den Anschlussport 6 der Verbindungsvorrichtung 1 und von dort der Patientenleitung zugeführt werden.

Das Gehäusebauteil 3 ist als im Wesentlichen zu einer Mittellängsachse M des Kanalabschnitts 5 koaxiales und rotationssymmetrisches Hohlkörperbauteil ausgeführt, das sowohl zur Außenseite, d.h. nach oben, als auch nach innen zum Fluidströmungsraum hin offen ist. Das Gehäusebauteil 3 bildet den Anschlussport 6, der an seinem Außenmantel im Bereich eines oberen Stirnendes mit einem Luer-Lock-Verbindungsabschnitt 10 versehen ist, um den Anschluss eines Luer-Lock-Anschlussteils wie insbesondere einer mit einem Luer-Lock-Konnektor versehenen Spritze zu ermöglichen. In dem Anschlussport 6 ist eine elastisch flexible Verschlussmembran 7 angeordnet, die vorzugsweise aus Silikon hergestellt ist. Die Verschlussmembran 7 bildet in unbelastetem Zustand einen dichten Abschluss des Anschlussports 6 sowohl zum Fluidströmungsraum als auch nach außen. Die Verschlussmembran 7 ist geschlitzt, so dass sie sich bei elastischer Deformation nach innen in den Fluidströmungsraum hinein öffnen kann. Die Verschlussmembran 7 ist zwischen dem Gehäusebauteil 3 und einem Stirnflansch des Grundgehäuses 2 eingeklemmt, der den Kanalabschnitt 5 nach oben begrenzt. Um den Stirnflansch koaxial herum ist eine ringförmige Aufnahmenut 12 in dem Grundgehäuse 2 vorgesehen, in die ein zylindrischer Steckflansch 11 des Gehäusebauteils 3 spielfrei einsteckbar ist. Die dichte Verbindung und Fixierung des Gehäusebauteils 3 relativ zum Grundgehäuse 2 in diesem eingesteckten Zustand erfolgt stoffschlüssig vorliegend durch Verklebung mittels eines Flüssigklebstoffs, der durch UV-Strahlung aushärtbar ist. Mithilfe von am Umfang des Gehäusebauteils 3 und des Grundgehäuses 2 vorgesehenen, zueinander komplementären Profilierungen 14, 15 wird eine Verdrehsicherung und eine positionsgenaue Ausrichtung des Gehäusebauteils 3 relativ zu dem Grundgehäuse 2 erzielt.

Auf einer der stirnseitigen Öffnung des Anschlussports 6 zugewandten Außenseite der Verschlussmembran 7 ist der Verschlussmembran 7 ein kolbenartiger Betätigungskörper 8 zugeordnet, der in dem Anschlussport 6 koaxial zur Mittellängsachse M verschiebbar gelagert ist. Der Betätigungskörper 8 weist einen zu der Mittellängsachse M koaxialen Durchtrittskanal 9 auf, der zu beiden Stirnseiten des Betätigungskörpers 8 hin offen ist.

Auch der Betätigungskörper 8 ist aus einem geeigneten Kunststoffmaterial hergestellt. Vorzugsweise weist auch das Kunststoffmaterial des Betätigungskörpers 8 eine hohe chemische Beständigkeit gegenüber hochdosierten medizinischen Wirkstoffen auf, die über den Anschlussport 6 der Verbindungsvorrichtung 1 zugeführt werden.

In dem Fluidströmungsraum der Verbindungsvorrichtung 1 ist in einem Mündungsbereich des ersten Kanalabschnitts 4 in den zweiten Kanalabschnitt 5 eine Strömungsleitwand 13 vorgesehen, die formstabil ausgeführt ist und einstückig aus dem Kunststoffmaterial des Grundgehäuses 2 in dem Fluidströmungsraum ausgeformt ist. Wie anhand der Fig. 1 und 4 gut erkennbar ist, ist die Strömungsleitwand 13 in einer Draufsicht V-artig in dem Kanalabschnitt 5 ausgerichtet, wobei die Strömungsleitwand 13 eine im Querschnitt - in der Draufsicht (Fig. 4) gemäß der Schnittlinie IV-IV in Fig. 3 gesehen - bogen- oder V-artig gekrümmte Wandung aufweist, die in die Innenwandung des Kanalabschnitts 5 im Bereich einer dem Mündungsabschnitt des Kanalabschnitts 4 gegenüberliegenden Innenwandungshälfte mündet. Hierdurch bildet die Strömungsleitwand 13 auf der dem Mündungsabschnitt des Kanalabschnittes 4 abgewandten Seite eine Rinne, die koaxial oder achsparallel zu der Mittellängsachse M verläuft. Die entsprechende Wandung der Strömungsleitwand 13 schließt an gegenüberliegenden Seiten über die gesamte Höhe des parallel zur Innenwandung des Kanalabschnitts 5 verlaufenden Wandungsabschnitts bündig und dicht mit der Innenwandung des Kanalabschnitts 5 ab. An einem unteren Endbereich der Strömungsleitwand 13 - auf die aufrechte Ausrichtung gemäß der Darstellung nach den Fig. 2 und 3 bezogen - weist die Strömungsleitwand 13 eine wannenartige Krümmung auf, die zu dem verbleibenden, gegenüberliegenden Innenwandungsabschnitt des Kanalabschnitts 5 erstreckt ist und dort in den Innenwandungsabschnitt des Kanalabschnitts 5 unterhalb des Mündungsbereichs des Kanalabschnitts 4 übergeht. Der so gebildete Wannenabschnitt erstreckt sich demzufolge kreisringabschnittsförmig unterhalb der aufrecht und parallel ausgerichteten V-förmigen Wandungsabschnitte der Strömungsleitwand 13 und bildet ein Auffangbecken für aus dem ersten Kanalabschnitt 4 in den Fluidströmungsraum einströmendes Fluid, wie anhand der punktierten Pfeilung gemäß den Fig. 2 und 3 dargestellt ist. Die Strömungsleitwand 13 bildet demzufolge für die durch den ersten Kanalabschnitt 4 geleitete Fluidströmung eine Umlenkfläche, durch die die Fluidströmung im Mündungsbereich zum Kanalabschnitt 5 durch die Strömungsleitwand 13 gestaut und in Richtung zur Verschlussmembran 7 umgelenkt wird. Bei geschlossener Verschlussmembran 7 strömt das Fluid über den als Teilbereich dienenden oberen Stirnrandbereich der Strömungsleitwand 13 hinweg und wird in den zweiten Kanalabschnitt 5 nach unten umgelenkt (Fig. 2). Damit ist gewährleistet, dass kein Totwasserbereich im Fluidströmungsraum entsteht.

Sobald durch das Ansetzen einer mit einem Luer-Konnektor verbundenen Spritze an den Anschlussport 6 der Betätigungskörper 8 in Richtung des Kanalabschnitts 5 nach unten verschoben wird, öffnet sich die flexible Verschlussmembran 7 zwangsläufig. Der Betätigungskörper 8 kann so weit nach unten verschoben werden, bis er mit seiner dem Kanalabschnitt 5 zugewandten unteren Stirnfläche, die den Durchtrittskanal 9 umgibt, auf dem oberen Stirnrandbereich der Strömungsleitwand 13 zur Anlage kommt oder unmittelbar oberhalb dieses Stirnrandbereichs beabstandet bleibt. Anhand der Fig. 4 ist erkennbar, dass die Ausrichtung und Anordnung der Krümmung der Strömungsleitwand 13 - im Querschnitt gesehen - so auf den Betätigungskörper 8 abgestimmt ist, dass der untere Stirnflächenbereich des Betätigungskörpers 8, der den Durchtrittskanal 9 umgibt, fluchtend oberhalb des Stirnrandbereichs der Strömungsleitwand 13 positioniert ist, sobald er in seine Öffnungsstellung verlagert ist. Dadurch fluchtet eine Innenwandung des Durchtrittskanals 9 des Betätigungskörpers 8 koaxial zur Mittellängsachse M mit einer dem Kanalabschnitt 5 zugewandten Wandungsseite der Strömungsleitwand 13. Die durch die punktierte Pfeilung längs der Mittellängsachse M in Fig. 3 dargestellte Fluidströmung von Fluid, das aus der Spritze über den Anschlussport 6 der Verbindungsvorrichtung 1 zugeführt wird, strömt daher durch den Durchtrittskanal 9 hindurch, an der Strömungsleitwand 13 im Bereich des Kanalabschnitts 5 entlang und zum unteren Anschluss des Kanalabschnitts 5. Dadurch, dass der untere Stirnrandbereich des Betätigungskörpers 8 mit dem oberen Stirnrandbereich der Strömungsleitwand 13 zumindest im Wesentlichen fluchtet, kann die durch den Anschlussport 6 über die Spritze zugeführte Fluidströmung bis auf vernachlässigbare Fluidmengen nicht in den ersten Kanalabschnitt 4 gelangen, so dass eine ungewünschte Aufwärtsströmung dieses über den Anschlussport 6 zugeführten Fluids vermieden wird. Denn Fluidmengen, die in Richtung des Kanalabschnittes 4 gelangen, treffen dort auf das Fluid des Kanalabschnittes 4 und die hierdurch aufgebaute Wassersäule, so dass allenfalls ein medizinisch vernachlässigbarer Rückfluss in diesen Kanalabschnitt 4 erfolgt.

Sobald das entsprechende Luer-Anschlussteil, vorliegend die Spritze, von dem Anschlussport 6 wieder entfernt wird, wird der Betätigungskörper 8 zwangsläufig wieder in seine Ruhestellung gemäß Fig. 2 zurückgeführt und die Verschlussmembran 7 stellt sich ebenfalls wieder in ihre dichte und geschlossene Ausgangslage gemäß Fig. 2 zurück.

Bei nicht dargestellten Ausführungsbeispielen der Erfindung sind die Strömungsleitwand 13 und der Betätigungskörper 8 und/oder die flexible Verschlussmembran 7 derart aufeinander abgestimmt, dass in der Öffnungsstellung der Verschlussanordnung der Betätigungskörper 8 oder die flexible Verschlussmembran 7 in den durch die Strömungsleitwand 13 gebildeten Rinnenbereich abschnittsweise eintauchen, so dass der Betätigungskörper und/oder die flexible Verschlussmembran neben der Strömungsleitwand 13 positioniert sind. Hierzu sind ein Stirnrandbereich der Strömungsleitwand 13 und ein Stirnflächenbereich des Betätigungskörpers zur Mittellängsachse M radial versetzt zueinander. Der Betätigungskörper 8 kann hierdurch mit seinem konisch zulaufenden Bereich abschnittsweise in den Rinnenbereich eintauchen.

## Patentansprüche

1. Verbindungsvorrichtung für ein Fluidsystem für medizinische Zwecke mit einem Grundgehäuse (2), das einen Fluidströmungsraum aufweist, der in verschiedenen Richtungen zu zwei offenen Anschlüssen hin führende Kanalabschnitte (4, 5) ausbildet, wobei die Anschlüsse zur Verbindung mit jeweils einer Fluidleitung oder einem Fluidspeicher vorgesehen sind, sowie mit einem in den Fluidströmungsraum mündenden Anschlussport (6), der mit einer Verschlussanordnung (7) versehen ist, und der einen Verbindungsabschnitt (10) zur Verbindung mit einem Anschlussteil umfasst, wobei der Fluidströmungsraum eine formstabile Strömungsleitwand (13) aufweist, die zwischen den beiden Kanalabschnitten (4, 5) im Fluidströmungsraum starr angeordnet ist, **dadurch gekennzeichnet, dass** die Strömungsleitwand (13) derart mit einem Stirnrand zu dem Anschlussport (6) hin frei abragt, dass die Verschlussanordnung in einer Öffnungsstellung mit dem Stirnrand der Strömungsleitwand (13) zusammenwirkt, um eine Fluidströmung vom Anschlussport (6) zumindest weitgehend zu lediglich einem Kanalabschnitt (5) zu erzielen, dass in dem Anschlussport (6) als Teil der Verschlussanordnung ein beweglicher Betätigungskörper (8) zum Öffnen einer flexiblen Verschlussmembran (7) in Abhängigkeit von einer Verbindung mit einem Luer-Anschlussteil gelagert ist, dass die Strömungsleitwand (13) derart in Richtung des Anschlussports abragt, dass der Betätigungskörper (8) und/oder die flexible Verschlussmembran (7) in der Öffnungsstellung der Verschlussanordnung mit Teilbereichen der Strömungsleitwand (13) derart in Anlage sind, dass eine in den Anschlussport (6) von dem Luer-Anschlussteil zugeführte Fluidströmung zumindest weitgehend in den Kanalabschnitt (5) strömt, und dass der Betätigungskörper (8) und/oder die flexible Verschlussmembran (7) in der Öffnungsstellung der Verschlussanordnung auf oder neben wenigstens einem Teilbereich der Strömungsleitwand (13) positioniert sind.

2. Verbindungsvorrichtung nach Anspruch 1, **dadurch gekennzeichnet, dass** der Verbindungsabschnitt (10) als Luer-Slip- oder Luer-Lock-Verbindungsabschnitt gestaltet ist.

3. Verbindungsvorrichtung nach Anspruch 1, **dadurch gekennzeichnet, dass** der Verbindungsabschnitt als Enteral Small Bore Connector ausbildet ist.

4. Verbindungsvorrichtung nach Anspruch 1, **dadurch gekennzeichnet, dass** die Strömungsleitwand (13) einstückig in dem Grundgehäuse (2) integriert ist.

5. Verbindungsvorrichtung nach einem der Ansprüche 1 bis 4, **dadurch gekennzeichnet, dass** die Strömungsleitwand (13) an dem freien Stirnrand über einen Teilbereich koaxial zu einer Mittellängsachse (M) des Anschlussports (6) im Querschnitt bogen- oder V-artig gekrümmt ist, wobei die Strömungsleitwand (13) in einem Mündungsbereich des einen Kanalabschnitts (4) in den anderen Kanalabschnitt (5) ausgeformt ist.

6. Verbindungsvorrichtung nach Anspruch 1, **dadurch gekennzeichnet, dass** eine Krümmung des freien Stirnrands der Strömungsleitwand (13) derart auf einen zu der Mittellängsachse (M) des Anschlussports (6) koaxialen Durchtrittskanal (9) des Betätigungskörpers (8) abgestimmt ist, dass eine derart gebildete Rinne der Strömungsleitwand (13) auf einer dem einen Kanalabschnitt (5) zugewandten Seite bei in seine Öffnungsstellung verlagertem Betätigungskörper (8) mit dem Durchtrittskanal (9) des Betätigungskörpers (8) fluchtet.

7. Verbindungsvorrichtung nach einem der vorhergehenden Ansprüche, **dadurch gekennzeichnet, dass** die Strömungsleitwand (13) für den einen Kanalabschnitt (4) als einen Fluidstrom umlenkende Strömungsleitfläche ausgeführt ist, die eine Fluidumlenkung in Richtung des Anschlussports (6) von aus dem einen Kanalabschnitt (4) in Richtung des anderen Kanalabschnitts (5) strömendem Fluid bewirkt.

8. Verbindungsvorrichtung nach dem Oberbegriff des Anspruchs 1 oder nach einem der vorhergehenden Ansprüche, **dadurch gekennzeichnet, dass** der Anschlussport (6) in einem zu dem Grundgehäuse (2) separat hergestellten Gehäusebauteil (3) vorgesehen ist, das aus einem inerteren Kunststoffmaterial hergestellt ist als ein Kunststoffmaterial des Grundgehäuses (2), um eine höhere chemische Beständigkeit als das Grundgehäuse (2) gegenüber medizinischen Wirkstoffen zu erzielen, die am Anschlussport (6) dem Fluidsystem zuführbar sind.

9. Verbindungsvorrichtung nach Anspruch 8, **dadurch gekennzeichnet, dass** das den Anschlussport (6) aufweisende Gehäusebauteil (3) stoffschlüssig, insbesondere durch Verklebung oder Verschweißung, mit dem Grundgehäuse (2) dicht verbunden ist.

## Claims

1. Connector device for a fluid system for medical purposes, with a main housing (2) which has a fluid flow chamber that forms channel portions (4, 5) leading in different directions towards two open attachments, wherein the attachments are provided for connection to in each case a fluid line or a fluid reservoir, and also with an attachment port (6) which opens into the fluid flow chamber and is provided with a closure arrangement (7) and which comprises a connecting portion (10) for connection to an attachment part, wherein the fluid flow chamber has a dimensionally stable flow-guiding wall (13) which is arranged rigidly between the two channel portions (4, 5) in the fluid flow chamber,
**characterized in that**
the flow-guiding wall (13) protrudes freely with a front edge towards the attachment port (6) in such a way that the closure arrangement, in an open position, interacts with the front edge of the flow-guiding wall (13) in order to obtain a flow of fluid from the attachment port (6) at least largely to only one channel portion (5),
**in that**, as part of the closure arrangement, a movable actuating body (8) for opening a flexible closure membrane (7) in accordance with a connection to a Luer attachment part is mounted in the attachment port (6),
**in that** the flow-guiding wall (13) protrudes in the direction of the attachment port in such a way that the actuating body (8) and/or the flexible closure membrane (7), in the open position of the closure arrangement, are in contact with partial areas of the flow-guiding wall (13) in such a way that a flow of fluid delivered into the attachment port (6) from the Luer attachment part flows at least largely into the channel portion (5), and
**in that** the actuating body (8) and/or the flexible closure membrane (7), in the open position of the closure arrangement, are positioned on or next to at least a partial area of the flow-guiding wall (13).

2. Connector device according to Claim 1, **characterized in that** the connecting portion (10) is designed as a Luer slip or Luer lock connecting portion.

3. Connector device according to Claim 1, **characterized in that** the connecting portion is designed as an enteral small bore connector.

4. Connector device according to Claim 1, **characterized in that** the flow-guiding wall (13) is integrated in one piece in the main housing (2).

5. Connector device according to any one of Claims 1 to 4, **characterized in that** the flow-guiding wall (13), at the free front edge, has an arc-shaped or V-shaped curvature in cross section over a partial area coaxial to a central longitudinal axis (M) of the attachment port (6), wherein the flow-guiding wall (13) is formed in a mouth region where one channel portion (4) leads into the other channel portion (5).

6. Connector device according to Claim 1, **characterized in that** a curvature of the free front edge of the flow-guiding wall (13) is adapted to a through-channel (9) of the actuating body (8), coaxial to the central longitudinal axis (M) of the attachment port (6), in such a way that a groove thereby formed in the flow-guiding wall (13), on a side facing towards one channel portion (5), is flush with the through-channel (9) of the actuating body (8) when the actuating body (8) is moved to its open position.

7. Connector device according to any one of the preceding claims, **characterized in that** the flow-guiding wall (13) is designed for one channel portion (4) as a flow-guiding surface which deflects a stream of fluid and which causes a deflection, in the direction of the attachment port (6), of fluid flowing out of one channel portion (4) in the direction of the other channel portion (5).

8. Connector device according to the preamble of Claim 1 or according to any one of the preceding claims, **characterized in that** the attachment port (6) is provided in a housing component (3) which is produced separately from the main housing (2) and which is produced from a more inert plastics material than a plastics material of the main housing (2), in order to achieve greater chemical resistance than the main housing (2) to medical active substances that can be delivered at the attachment port (6) to the fluid system.

9. Connector device according to Claim 8, **characterized in that** the housing component (3) having the attachment port (6) is connected tightly to the main housing (2) by cohesive bonding, in particular by gluing or welding.

## Revendications

1. Dispositif de connexion d'un système fluidique à des fins médicales avec un boîtier de base (2), qui présente une chambre d'écoulement de fluide, qui forme des portions de canal (4, 5) menant dans différentes directions à des raccords ouverts, dans lequel les raccords sont prévus pour la connexion respectivement avec une conduite de fluide ou un réservoir de fluide, ainsi qu'avec un orifice de raccord (6) débouchant dans la chambre d'écoulement de fluide, qui est doté d'un dispositif de fermeture (7), et qui comprend une portion de connexion (10) pour la connexion avec une partie de raccord, dans lequel la chambre d'écoulement de fluide présente une paroi de guidage d'écoulement de forme stable (13), qui est disposée de façon rigide dans la chambre d'écoulement de fluide entre les deux portions de canal (4, 5), **caractérisé en ce que** la paroi de guidage d'écoulement (13) est saillante librement avec un bord frontal en direction de l'orifice de raccord (6), de telle manière que le dispositif de fermeture coopère dans une position ouverte avec le bord frontal de la paroi de guidage d'écoulement (13) pour produire un écoulement de fluide de l'orifice de raccord (6) au moins largement uniquement vers une portion de canal (5), **en ce qu'**un corps d'actionnement mobile (8) est monté dans l'orifice de raccord (6) en tant que partie du dispositif de fermeture, pour ouvrir une membrane de fermeture flexible (7) en fonction d'une connexion avec une partie de raccord Luer, **en ce que** la paroi de guidage d'écoulement (13) est saillante en direction de l'orifice de raccord, de telle manière que le corps d'actionnement (8) et/ou la membrane de fermeture flexible (7) s'appliquent dans la position ouverte du dispositif de fermeture sur des régions partielles de la paroi de guidage d'écoulement (13), de telle manière qu'un écoulement de fluide envoyé dans l'orifice de raccord (6) par la partie de raccord Luer s'écoule au moins largement dans la portion de canal (5), et **en ce que** le corps d'actionnement (8) et/ou la membrane de fermeture flexible (7) sont positionnés, dans la position ouverte du dispositif de fermeture, sur ou à côté d'au moins une région partielle de la paroi de guidage d'écoulement (13).

2. Dispositif de connexion selon la revendication 1, **caractérisé en ce que** la partie de connexion (10) est formée par une partie de connexion Luer-Slip ou Luer-Lock.

3. Dispositif de connexion selon la revendication 1, **caractérisé en ce que** la partie de connexion est formée par un raccord de petite taille à usage entéral.

4. Dispositif de connexion selon la revendication 1, **caractérisé en ce que** la paroi de guidage d'écoulement (13) est intégrée d'une pièce dans le boîtier de base (2) .

5. Dispositif de connexion selon l'une quelconque des revendications 1 à 4, **caractérisé en ce que** la paroi de guidage d'écoulement (13) est incurvée en section transversale en forme d'arc ou de V au bord frontal libre sur une région partielle coaxialement à un axe central longitudinal (M) de l'orifice de raccord (6), dans lequel la paroi de guidage d'écoulement (13) est formée dans une région d'embouchure de la première portion de canal (4) dans l'autre portion de canal (5).

6. Dispositif de connexion selon la revendication 1, **caractérisé en ce qu'**une courbure du bord frontal libre de la paroi de guidage d'écoulement (13) est accordée à un canal de passage (9) du corps d'actionnement (8) coaxial à l'axe central longitudinal (M) de l'orifice de raccord (6), de telle manière qu'une goulotte ainsi formée de la paroi de guidage d'écoulement (13) soit alignée sur un côté tourné vers la première portion de canal (5) avec le canal de passage (9) du corps d'actionnement (8) lorsque le corps d'actionnement (8) est déplacé dans sa position ouverte.

7. Dispositif de connexion selon l'une quelconque des revendications précédentes, **caractérisé en ce que** la paroi de guidage d'écoulement (13) est réalisée pour une première portion de canal (4) sous la forme d'une face de guidage d'écoulement déviant un flux de fluide, qui provoque une déviation de fluide en direction de l'orifice de raccord (6) de fluide qui s'écoule hors de la première portion de canal (4) en direction de l'autre portion de canal (5).

8. Dispositif de connexion selon le préambule de la revendication 1 ou selon l'une quelconque des revendications précédentes, **caractérisé en ce que** l'orifice de raccord (6) est prévu dans un élément de boîtier (3) fabriqué séparément du boîtier de base (2), qui est fabriqué en un matériau à base de matière plastique plus inerte qu'un matériau à base de matière plastique du boîtier de base (2), afin d'obtenir une résistance chimique plus élevée que le boîtier de base (2) à l'égard de substances actives médicales, qui peuvent être fournies au système fluidique à l'orifice de raccord (6).

9. Dispositif de connexion selon la revendication 8, **caractérisé en ce que** l'élément de boîtier (3) présentant l'orifice de raccord (6) est matériellement assemblé de façon étanche, en particulier par collage ou soudage, au boîtier de base (2).
